Europäisches Patentamt

European Patent Office

Office européen des brevets

(11) Veröffentlichungsnummer: **0 465 842 A2**

# (12) EUROPÄISCHE PATENTANMELDUNG

(21) Anmeldenummer: **91109411.8**

(22) Anmeldetag: **08.06.91**

(51) Int. Cl.⁵: **G01N 33/36**, D02G 1/16

(30) Priorität: **15.06.90 DE 4019106**

(43) Veröffentlichungstag der Anmeldung:
**15.01.92 Patentblatt 92/03**

(84) Benannte Vertragsstaaten:
**BE CH DE DK ES FR GB GR IT LI LU NL**

(71) Anmelder: **HOECHST AKTIENGESELLSCHAFT**
**Postfach 80 03 20**
**W-6230 Frankfurt am Main 80(DE)**

(72) Erfinder: **Bönigk, Burkhard, Dr.**

**Haferfeldring 14**
**W-8901 Königsbrun(DE)**
Erfinder: **Jacob, Ingolf, Dr.**
**Nibelungering 35**
**W-8933 Untermeitingen(DE)**
Erfinder: **Kretschmer, Peter**
**Friedensstrasse 11**
**W-8903 Bobingen(DE)**
Erfinder: **Schneider, August, Dr.**
**Lärchenstrasse 1**
**W-8934 Grossaitingen(DE)**

(54) **Verfahren und Vorrichtung zum Messen des Verwirbelungszustands eines Multifilamentgarnes.**

(57) Beschrieben werden ein Verfahren und eine Vorrichtung zum Messen des Verwirbelungszustandes eines verwirbelten Multifilamentgarnes. Das verwirbelte Garn wird durch eine selbstfördernde Fluiddüse (Luftdüse) bewegt. Das Garn füllt den Düsenkanal weitgehend aus, während es in praktisch zugspannungsfreiem Zustand aus der Düse austritt. Aufgrund des am Düsenausgang auftretenden Druckunterschiedes werden nicht geschlossene Garnzonen explosionsartig, ballonförmig aufgeweitet. Im aufgeweiteten Bereich des Garns ist eine Sensoreinrichtung, vorzugsweise eine elektronische Kamera, angeordnet, die die durch die Garnfilamente hervorgerufenen Helligkeitsunterschiede oder Lichtbeugungsphänomene erfaßt und in Abhängigkeit hiervon ein Signal an eine Signalverarbeitungseinrichtung abgibt. Das erfindungsgemäße Meßprinzip ermöglicht sehr präzise Aussagen über die Art der Verwirbelung (punktförmig oder kontinuierlich) wie auch über die Geometrie der Verwirbelung (z.B. Anzahl und Abstand von Verwirbelungsknoten im Falle einer punktförmigen Verwirbelung).

Fig. 1

EP 0 465 842 A2

Die Erfindung betrifft ein Verfahren und eine Vorrichtung zum Messen des Verwirbelungszustandes eines verwirbelten Multifilamentgarnes, das ein Einzel- oder Mehrkomponentengarn sein kann.

Die in der Praxis eingesetzten Meßmethoden wie der Nadeltest bzw. der Interlace--Counter erweisen sich bei dem heute erreichten Stand der Verwirbelung zunehmend als unbefriedigend. So erlaubt z. B. der Nadeltest bei sehr hoher Verwirbelungsdichte keine sinnvolle Aussage mehr, und bei einer kontinuierlichen Verwirbelung versagt er völlig.

Aus der DE-OS 28 39 439 sind ein Verfahren und eine Vorrichtung zum Messen des Verwirbelungszustandes eines verwirbelten Multifilamentgarnes bekannt, bei dem offene Zonen des verwirbelten Garns aufgeweitet und berührungslos abgefühlt werden. Zum Aufweiten des Garnes wird in diesem Fall das Garn über eine gewölbte durchsichtige Platte geführt, und die hierdurch bedingten Formänderungen werden mittels einer Photodetektoranordnung erfaßt. Dieses Meßprinzip erlaubt nur einen diskontinuierlichen Betrieb. In der EP 340 600 wird das zu überwachende Garn auf einem mit Unterdruck beaufschlagten Förderband abgelegt und dann ebenfalls mittels eines optischen Sensors abgefühlt. Nachteilig bei beiden vorbekannten Verfahren ist, daß das Garn während des Meßvorganges an mechanischen Teilen anliegt und hierbei verformt wird, was zu einer entsprechenden Verfälschung des Meßsignales führen kann.

Der Erfindung liegt die Aufgabe zugrunde, ein Verfahren und eine Vorrichtung zum Messen des Verwirbelungszustandes eines verwirbelten Multifilamentgarnes anzugeben, die eine möglichst genaue und umfassende Charakterisierung des Verwirbelungszustandes des Garnes erlauben.

Zur Lösung dieser Aufgabe sind das erfindungsgemäße Verfahren und die erfindungsgemäße Vorrichtung durch die in den Ansprüchen 1 und 16 angegebenen Merkmale gekennzeichnet.

Erfindungsgemäß werden die offenen Zonen des Garns durch einen Fluidstrom, insbesondere einen Luftstrom aufgeweitet. Das Aufweiten wie auch das berührungsfreie Abfühlen erfolgen in einem Bereich, in dem das Garn ausschließlich von Fluid, insbesondere Luft umgeben ist. Im Gegensatz zu dem eingangs diskutierten Stand der Technik liegt somit das Garn im Meßbereich an keinen mechanischen Teilen an. Es kann sich daher in sämtlichen Freiheitsgraden entfalten, abgesehen natürlich von der durch das Garn selbst bedingten Beschränkung.

Zum Aufweiten wird das Garn vorzugsweise durch eine Fluiddüse bewegt. Die Fluiddüse kann eine mit Luft betriebene Verwirbelungsdüse sein, wie sie z.B. aus der US-PS 32 73 328 bekannt ist und dort zum Verwirbeln einer oder mehrerer Garnkomponenten verwendet wird. Im Gegensatz zu diesem Stand der Technik wird bei dem erfindungsgemäßen Verfahren das Garn in im wesentlichen zugspannungsfreiem Zustand aus der Fluiddüse abgeführt, so daß sich die nicht geschlossenen Zonen des Garnes am Düsenausgang explosionsartig ballonförmig aufweiten. Damit die Fluiddüse selbstfördernd ist, sollte das Garn in der Fluiddüse mit einem Fluidstrahl (Luftstrahl) beaufschlagt werden, der eine in Richtung der Düsenachse verlaufende Bewegungskomponente hat.

Durch die am Düsenausgang erfolgende "kontrollierte Explosion" werden somit die offenen (d.h. nicht verwirbelten) Garnzonen und ggf. auch leicht verklebte und/oder verklemmte Garnzonen ballonförmig "aufgesprengt". Auf diese Weise wird der Verwirbelungszustand (Fadenschluß) des Garns besonders ausgeprägt, was ein präzises Erfassen des Verwirbelungszustandes erlaubt. Da ferner das aufgeweitete Garn an keinen mechanischen Teilen anliegt, ist eine Verfälschung des Meßsignales durch eine andernfalls unvermeidliche Verformung des Garnes ausgeschlossen.

Die Erfindung erlaubt nicht nur eine präzise, sondern auch umfassende Charakterisierung des Verwirbelungszustandes. So läßt sich mit der Erfindung feststellen, ob es sich um eine punktförmige oder kontinuierliche Verwirbelung handelt. Auch kann der Verwirbelungszustand zweier oder mehrerer Garnkomponenten ("ein- oder mehrzöpfiger" Fadenschluß) festgestelltwerden. Bei punktförmiger Verwirbelunglassen sich die Verwirbelungsfehlstellen, die Anzahl der Verwirbelungspunkte pro Garnlänge, die mittlere Länge der verwirbelten wie auch unverwirbelten Garnzonen ermitteln. Ferner erlaubt die Erfindung eine Aussage über die Fadenschlußkraft.

Wie bereits erwähnt, sollte das Garn aus der Fluiddüse nicht unter Zugspannung abgezogen, sondern von der selbstfördernden Fluiddüse gewissermaßen nach außen gedrückt werden. Die "kontrollierte Garnexplosion" am Düsenausgang ist um so ausgeprägter, je größer der Druckabfall beim Austritt ist. Zu diesem Zweck sollte das Garn durch die Fluiddüse mit einem so hohen Füllgrad bewegt werden, daß das Garn den Düsenkanal praktisch vollständig ausfüllt. Hierdurch wird außerdem eine erneute Verwirbelung des Garns in der Fluiddüse vermieden.

Das Abfühlen des aufgeweiteten Garnes kann auf optischem Wege oder mittels Ultraschall erfolgen. Vorzugsweise ist vorgesehen, daß das Garn im aufgeweiteten Bereich von einer elektrischen Kamera, vorzugsweise eine Zeilenkamera überwacht wird.

Vorzugsweise ist auf der der Kamera abgewandten Seite des Garnes eine Beleuchtungsquelle bzw. ein Laser zum Beleuchten des Garnes angeordnet. Die in der Zeilenkamera vorgesehene Diodenzeile erfaßt hierbei die durch die Garnfilamente hervorgerufenen Helligkeitsunterschiede bzw. Beugungsphänomene in ihrem Meßfeld. Das resultierende Meßsignal wird an eine Signalverarbeitungseinrichtung weitergegeben, die z.B. einen Controller aufweist, welcher das in Analogform vorliegende Meßsignal digitalisiert und an einen Rechner zur Auswertung abgibt.

Da das erfindungsgemäße Meßprinzip berührungslos arbeitet und daher von Massenträgheiten unabhängig ist, können extrem hohe Prüfgeschwindigkeiten erzielt werden. Hieraus folgt eine drastische Erhöhung der statistischen Sicherheit. So können z.B. bei dem erfindungsgemäßen Verfahren einige 100 m Garn in einer Zeit geprüft werden, in der beim Nadeltest oder mit dem Interlace-Counter nur 30 cm bzw. 5 m geprüft werden können.

Darüberhinaus erlaubt die Erfindung eine Messung des Garns im on-line-Betrieb, was bei den bisher bekannten Verfahren nicht möglich ist.

In weiterer Ausgestaltung der Erfindung ist vorgesehen, daß zum räumlichen Messen des Garns das Abfühlen in zwei aufeinander senkrecht stehenden Richtungen erfolgt. Hierzu können beispielsweise zwei aufeinander senkrecht stehende elektronische Kameras verwendet werden.

Ferner ist es besonders vorteilhaft, das erfindungsgemäße Verfahren so zu führen daß das Garn vor seinem Einlauf in den Fluidstrom belastet wird. Die Belastung des Garns besteht zweckmäßigerweise z.B. aus einem elastischen Dehnen des Garns oder aus einem Umlenken um scharfe Kanten. Für andere Fragestellungen wird die Belastung als eine Wechselbelastung des Garns ausgeführt.

Vorzugsweise sind hierzu vor der Fluiddüse zwei in Laufrichtung beabstandete regelbare Fördereinrichtungen (z.B. Galetten) angeordnet, durch die dem in die Fluiddüse einzuführenden Garn eine wählbare Spannung verliehen werden kann. Dies erlaubt eine elastische Dehnung des Garns, um somit die Belastung der Verwirbelung z.B. auf Web-, Wirk- oder Strickmaschinen näherungsweise zu simulieren. Durch Zu- bzw. Abschalten der Verdehnung läßt sich in nur einem einzigen Prüfdurchgang der Unterschied zwischen jungfräulich verwirbeltem und elastisch verdehntem Garn feststellen. Hierdurch erhält man eine sonst kaum zugängliche Information über die Fadenschlußkraft der Verwirbelung.

Mit besonderem Vorteil kann das erfindungsgemäße Verfahren auch zum Messen eines Mehrkomponentengarnes eingesetzt werden, um unvollkommen verwirbelte Zonen des Garns identifizieren und orten zu können.

Die vorliegende Erfindung betrifft auch eine Vorrichtung zur Durchführung des erfindungsgemäßen Verfahrens zum Messen des Verwirbelungszustandes eines verwirbelten Multifilamentgarnes.

Diese Vorrichtung weist eine Einrichtung zum Aufweiten offener Zonen des verwirbelten Garns, eine Sensoreinrichtung, die das aufgeweitete Garn berührungslos abfühlt und in Abhängigkeit hiervon ein Meßsignal erzeugt, und eine Signalverarbeitungseinrichtung für das Meßsignal auf, wobei die Einrichtung zum Aufweiten offener Zonen des Garns aus einer Fluiddüse 8 besteht, durch die das verwirbelte Garn 2 geführt wird, und daß die Sensoreinrichtung 10 im Bereich des Düsenausgangs angeordnet ist.

Die Fluiddüse 8 weist mindestens einen in den Düsenkanal 11 mündenden Blaskanal 13 auf, der zur Düsenlängsrichtung schräg verläuft, so daß sie selbstfördernd ist.

Hinter der Fluiddüse 8 ist eine regelbare Fördereinrichtung 12 angeordnet, die das Garn 2 in im wesentlichen zugspanungsfreiem Zustand aus der Fluiddüse 8 abführt.

Vorzugsweise sind auch vor der Fluiddüse 8 zwei in Laufrichtung beabstandete regelbare Fördereinrichtungen 4, 6 angeordnet, die dem in die Fluiddüse 8 einzuführenden Garn 2 eine wählbare Spannung verleihen.

Die Sensoreinrichtung 10 weist vorzugsweise eine elektronische Kamera, insbesondere eine Zeilenkamera 22 auf, die das Garn 2 im aufgeweiteten Bereich optisch abfühlt. Besondere meßtechnische Vorteile bietet eine Sensoreinrichtung, die eine zweite elektronische Kamera aufweist, deren optische Achse auf der der ersten Kamera senkrecht steht.

Zweckmäßigerweise ist auf der der Kamera 22 abgewandten Seite des Garns 2 eine Beleuchtungsquelle 20 oder ein Laser zum Beleuchten des Garns 2 angeordnet.

Besonders bevorzugt ist eine erfindungsgemäße Vorrichtung, deren Signalverarbeitungseinrichtung einen Controller 26 aufweist, der das von der Sensoreinrichtung 10 empfangene analoge Meßsignal digitalisiert und an einen Rechner 28 zur Auswertung abgibt.

Anhand der Zeichnungen wird ein Ausführungsbeispiel der Erfindung erläutert. Es zeigt:

Figur 1 eine schematische Ansicht einer Vorrichtung zum Messen des Verwirbelungszustandes eines verwirbelten Multifilamentgarnes;

Figuren 2 bis 5 Diagramme, die Beispiele für das von der Vorrichtung der Figur 1 erzeugte Signal darstellen.

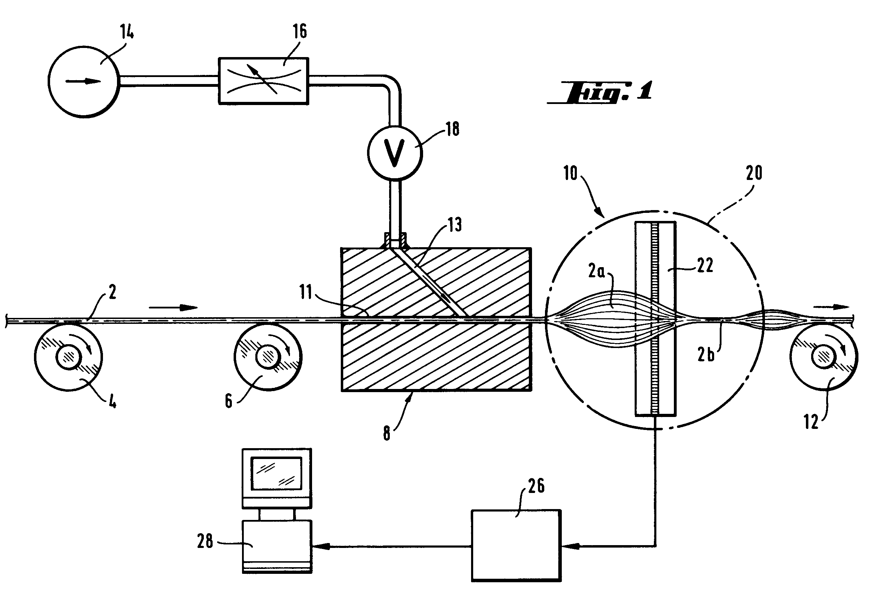

Die in Figur 1 schematisch gezeigte Vorrichtung besitzt zwei als Galettensystem ausgebildete Fördereinrichtungen 4, 6, eine Fluiddüse 8, eine Sensoreinrichtung 10 sowie eine ebenfalls als Galettensystem ausgebildete Fördereinrichtung 12, die das zu untersuchende verwirbelte Multifilamentgarn 2 nacheinander durchläuft.

Die Fluiddüse 8 ist von ihrer Bauart her eine Luftverwirbelungsdüse mit einem das Garn 2 aufnehmenden Düsenkanal 11 und einem oder mehreren in den Düsenkanal 11 mündenden Blaskanälen 13. Die Blaskanäle 13 werden von einer Druckluftquelle 14 über eine Druckmindereinrichtung 16 und ein Absperrventil 18 mit Druckluft versorgt. Die Blaskanäle 13 sind unter einem spitzen Winkel (z.B. 45°) zu dem Düsenkanal 11 angeordnet, so daß die Blasluft mit einer horizontalen Bewegungskomponente in den Düsenkanal 11 einströmt und dadurch die Förderung des Garns 2 durch die Fluiddüse 8 bewirkt.

Das Garn 2 braucht somit am Düsenausgang nicht gezogen zu werden. Die Fördereinrichtung 12 dient lediglich dazu, das andernfalls peitschende Garn 2 in eine uniaxiale Laufrichtung zu zwingen. Die Geschwindigkeit der Fördereinrichtung 12 wird hierbei über eine (nicht gezeigte) Wägezelle so geregelt, daß die Zugspannung des Garnes 2 am Düsenaustritt praktisch Null ist.

Der Durchmesser des Garns 2 und der Durchmesser des Düsenkanals 11 werden so gewählt, daß das Garn 2 den Düsenkanal 11 praktisch vollständig ausfüllt. Auf diese Weise wird eine Nachverwirbelung des Garns 2 vermieden. Außerdem entsteht dadurch ein besonders ausgeprägter Druckabfall am Düsenaustritt.

Aufgrund dieses Druckabfalls kommt es am Düsenaustritt zu einem - schematisch dargestellten - explosionsartigen ballonartigen Aufweiten des Garns 2, das im dargestellten Ausführungsbeispiel ein punktverwirbeltes Garn mit offenen Zonen 2a und geschlossenen Zonen 2b ist.

In dem Bereich, in dem die expandierende Luft die offenen Zonen 2a aufweitet, ist die Sensoreinrichtung 10 angeordnet, die eine Beleuchtungsquelle 20 sowie eine elektronische Kamera 22 in Form einer Zeilenkamera aufweist. Die Beleuchtungsquelle 20 und die Zeilenkamera 22 sind auf voneinander abgewandten Seiten des Garns 2 angeordnet, wobei die Diodenleiste der Zeilenkamera 22 die Helligkeitsunterschiede zwischen den das Licht durchlassenden Stellen und den von den Filamenten des Garnes 2 verdunkelten Stellen erfaßt.

Das Auflösungsvermögen der Zeilenkamera 22 sollte so gewählt werden, daß sie die Einzelfilamente eines Garnes feinsten Titers (z.B. 0,5 dtex) erfassen kann. Die Länge der Diodenzeile der Zeilenkamera 22 beträgt z.B. 26 mm, und auf dieser Strecke sind 2048 bildauflösende Elemente in einer Reihe angeordnet. Der Abstand zwischen diesen Elementen beträgt jeweils ca. 13 $\mu$m. Die minimale Belichtungszeit ist 500 $\mu$s. Die Zeilenkamera 22 erzeugt ein analoges Meßsignal, das einem Controller 26 zugeführt wird. Der mit einem A/D-Wandler versehene Controller 26 digitalisiert das Meßsignal und gibt es an einen schnellen Prozeßrechner 28 ab. In dem Prozeßrechner 28 erfolgt dann die Auswertung des Meßsignales, das auch am Bildschirm optisch dargestellt werden kann.

Um den Verwirbelungszustand des Garnes 2 räumlich erfassen zu können, kann zusätzlich zu der Sensoreinrichtung 10 eine um 90° verdrehte zweite Sensoreinrichtung vorgesehen werden. Eine derartige räumliche Erfassung des Garnes wäre insbesondere bei nicht rotationssymmetrischen Garnen bzw. Garnzonen von Bedeutung.

Die vor der Fluiddüse 8 angeordneten Fördereinrichtungen 4, 6 sind in ihrer Geschwindigkeit regelbar, so daß mit ihnen das Garn 2 elastisch gedehnt werden kann. Auf diese Weise läßt sich die Belastung des verwirbelten Garnes simulieren, der es bei der späteren Verwendung auf einer Web-, Wirk- oder Strickmaschine ausgesetzt ist. Außerdem kann in nur einem Prüfdurchgang - durch Zu- bzw. Abschalten der Verstreckung - der Unterschied zwischen spannungsfreiem und elastisch verdehntem Garn ermittelt werden, was eine Aussage über die Fadenschlußkraft erlaubt.

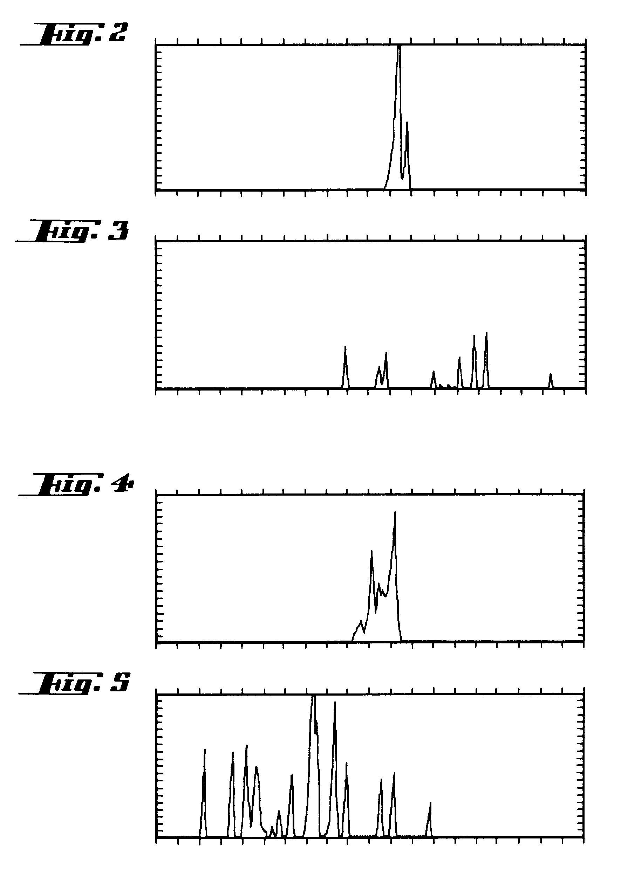

Mit der in Figur 1 gezeigten Vorrichtung lassen sich mit hoher Genauigkeit die offenen und geschlossenen Zonen 2a, b eines punktverwirbelten Multifilamentgarnes 2 erfassen. So stellt z.B. das im Diagramm der Figur 2 gezeigte Signal eine geschlossene Zone eines punktverwirbelten Garnes 50f40 dar, während das Diagramm der Figur 3 eine offene Zone des gleichen Garnes wiedergibt.

Mit der Vorrichtung der Figur 1 lasssen sich glatte wie auch texturierte Garne prüfen. Das in Figur 4 gezeigte Signal stellt ein geschlossenes texturiertes Garn 50f40 dar, während das Signal der Figur 5 für ein unverwirbeltes geöffnetes glattes Garn 50f40 gilt.

Die Prüfung mit der Vorrichtung nach Figur 1 kann im on-line-Betrieb erfolgen. Bei einer Prüfgeschwindigkeit von 800m/min würden 6,7 mm Garn pro 500 $\mu$s die Diodenzeile der Zeilenkamera kreuzen. Ein Stroboskop oder eine rotierende Schlitzscheibe (nicht gezeigt) kann diese Belichtungszeit noch verkürzen.

**Patentansprüche**

1. Verfahren zum Messen des Verwirbelungszustandes eines verwirbelten Multifilamentgarnes, bei dem offene Zonen des verwirbelten Garnes aufgeweitet und berührungslos abgefühlt werden, dadurch gekennzeichnet, daß das Aufweiten durch einen Fluidstrom erfolgt und das Aufweiten und Abfühlen in einem Bereich durchgeführt werden, in dem das Garn ausschließlich von Fluid umgeben ist.

2. Verfahren nach Anspruch 1, dadurch gekennzeichnet, daß das verwirbelte Garn durch eine Fluiddüse bewegt und in im wesentlichen zugspannungsfreiem Zustand aus der Fluiddüse abgeführt wird, so daß sich die offenen Garnzonen am Düsenausgang schlagartig aufweiten.

3. Verfahren nach Anspruch 2, dadurch gekennzeichnet, daß das Garn in der Fluiddüse mit einem Fluidstrahl beaufschlagt wird, der eine in Richtung der Düsenachse verlaufende Bewegungskomponente hat.

4. Verfahren nach Anspruch 2 oder 3, dadurch gekennzeichnet, daß das Garn durch die Fluiddüse mit einem so hohen Füllgrad bewegt wird, daß das Garn den Düsenkanal praktisch vollständig ausfüllt.

5. Verfahren nach einem der vorhergehenden Ansprüche, dadurch gekennzeichnet, daß das Abfühlen auf optischem Wege oder mittels Ultraschall erfolgt.

6. Verfahren nach Anspruch 5, dadurch gekennzeichnet, daß das Garn im aufgeweiteten Bereich beleuchtet und die dort auftretenden Helligkeitsunterschiede durch eine elektronische Kamera, vorzugsweise eine Zeilenkamera erfaßt werden.

7. Verfahren nach Anspruch 5, dadurch gekennzeichnet, daß das Garn im aufgeweiteten Bereich mit einem Laser angestrahlt und die dort auftretenden Lichtbeugungsphänomene durch eine elektronische Kamera, vorzugsweise eine Zeilenkamera erfaßt werden.

8. Verfahren nach einem der vorhergehenden Ansprüche, dadurch gekennzeichnet, daß zum räumlichen Messen des Garns das Abfühlen in zwei aufeinander senkrecht stehenden Richtungen erfolgt.

9. Verfahren nach einem der vorhergehenden Ansprüche, dadurch gekennzeichnet, daß das Garn vor seinem Einlauf in den Fluidstrom belastet wird.

10. Verfahren nach Anspruch 9, dadurch gekennzeichnet, daß die Belastung aus einem elastischen Dehnen des Garns besteht.

11. Verfahren nach Anspruch 9 oder 10, dadurch gekennzeichnet, daß die Belastung aus einem Umlenken um scharfe Kanten besteht.

12. Verfahren nach einem der Ansprüche 9 bis 11, dadurch gekennzeichnet, daß die Belastung aus einer Wechselbelastung besteht.

13. Verfahren nach einem der Ansprüche 9 bis 12, dadurch gekennzeichnet, daß während eines einzigen Prüfdurchganges durch zu- oder Abschalten einer wählbaren Belastung der Unterschied zwischen jungfäulich verwirbeltem Garn und belastetem verwirbeltem Garn ermittelt wird, um eine Aussage über die Fadenschlußkraft der Verwirbelung zu erhalten.

14. Verfahren nach einem der vorhergehenden Ansprüche, dadurch gekennzeichnet, daß es zum Messen eines Mehrkomponentengarnes verwendet wird, um unvollkommen verwirbelte Zonen des Garns identifizieren und orten zu können.

15. Verfahren nach einem der vorhergehenden Ansprüche, dadurch gekennzeichnet, daß es im on-line-Betrieb durchgeführt wird.

16. Vorrichtung zum Messen des Verwirbelungszustandes eines verwirbelten Multifilamentgarnes, insbesondere zum Durchführen des Verfahrens einer der vorhergehenden Ansprüche, mit einer Einrichtung zum Aufweiten offener Zonen des verwirbelten Garns, einer Sensoreinrichtung, die das aufgeweitete Garn berührungslos abfühlt und in Abhängigkeit hiervon ein Meßsignal erzeugt, und einer Signalverarbeitungseinrichtung für das Meßsignal, dadurch gekennzeichnet, daß die Einrichtung zum Aufweiten offener Zonen des Garns aus einer Fluiddüse (8) besteht, durch die das verwirbelte Garn (2) geführt wird, und daß die Sensoreinrichtung (10) im Bereich des Düsenausgangs angeordnet ist.

17. Vorrichtung nach Anspruch 16, dadurch gekennzeichnet, daß die Fluiddüse (8) mindestens einen in den Düsenkanal (11) mündenden Blaskanal (13) aufweist, der zur Düsenlängsrichtung schräg verläuft, so daß die Fluiddüse (8) selbstfördernd ist.

18. Vorrichtung nach Anspruch 16 oder 17, dadurch gekennzeichnet, daß hinter der Fluiddüse (8) eine regelbare Fördereinrichtung (12) angordnet ist, die das Garn (2) in im wesentlichen zugspanungsfreiem Zustand aus der Fluiddüse (8) abführt.

19. Vorrichtung nach einem der Ansprüche 16 bis 18, dadurch gekennzeichnet, daß vor der Fluiddüse (8) zwei in Laufrichtung beabstandete regelbare Fördereinrichtungen (4, 6) angeordnet sind, die dem in die Fluiddüse (8) einzuführenden Garn (2) eine wählbare Spannung verleihen.

**20.** Vorrichtung nach einem der Ansprüche 16 bis 19, dadurch gekennzeichnet, daß die Sensoreinrichtung (10) eine elektronische Kamera, insbesondere eine Zeilenkamera (22) aufweist, die das Garn (2) im aufgeweiteten Bereich optisch abfühlt.

**21.** Vorrichtung nach Anspruch 20, dadurch gekennzeichnet, daß auf der der Kamera (22) abgewandten Seite des Garns (2) eine Beleuchtungsquelle (20) oder ein Laser zum Beleuchten des Garns (2) angeordnet ist.

**22.** Vorrichtung nach Anspruch 20 oder 21, dadurch gekennzeichnet, daß die Sensoreinrichtung eine zweite elektronische Kamera aufweist, deren optische Achse auf der der ersten Kamera senkrecht steht.

**23.** Vorrichtung nach einem der Ansprüche 16 bis 22, dadurch gekennzeichnet, daß die Signalverarbeitungseinrichtung einen Controller (26) aufweist, der das von der Sensoreinrichtung (10) empfangene analoge Meßsignal digitalisiert und an einen Rechner (28) zur Auswertung abgibt.

Fig. 1

EP 0 465 842 A2

**Fig. 2**

**Fig. 3**

**Fig. 4**

**Fig. 5**